# EUROPEAN PATENT APPLICATION

(11) **EP 2 733 632 A2**
(43) Date of publication of application: **21.05.2014**
(21) Application number: 13189522.9
(22) Date of filing: 21.10.2013
(51) Int. Cl.: G06F 19/00

(54) **Apparatus and methods for remote disease management**

(30) Priority: 16.11.2012 KR 20120130049
(71) Applicant: Samsung Electronics Co., Ltd, Gyeonggi-do 443-742 (KR)
(72) Inventor: Bae, Sang Kon, 446-712 Gyeonggi-do (KR); Shin, Kun Soo, 446-712 Gyeonggi-do (KR); Kang, Jae Min, 446-712 Gyeonggi-do (KR); Ko, Byung Hoon, 446-712 Gyeonggi-do (KR); Kim, Youn Ho, 446-712 Gyeonggi-do (KR); Park, Kun Kook, 446-712 Gyeonggi-do (KR)
(74) Representative: Grünecker, Kinkeldey, Stockmair & Schwanhäusser

(57) **Abstract**

An apparatus and method for remotely managing a disease. The apparatus includes a sensor having a first determining unit for determining whether a measured biosignal has a normal waveform, and a transmitter for transmitting the biosignal to a server when the biosignal is determined to have an abnormal waveform.

## Description

### BACKGROUND

### 1. Field

The following description relates to an apparatus and method for remotely managing a patient with a cardiac disease, including chronic cardiac diseases, and accurately managing arrhythmia of a patient.

### 2. Description of Related Art

Typically, an abnormality may be detected in a heart of a patient with a cardiac disease by monitoring a cardiac electrical signal, that is, an electrocardiogram (ECG). In a general hospital, an ECG test may be performed on-site for a period of about two or three minutes. As a result, the condition of the patient may be monitored, and whether an abnormality exists may be diagnosed. Typically, an accurate ECG test for a patient experiencing an abnormality should result in an abnormal ECG being represented.

However, since a patient with a cardiac disease may experience an abnormality at different periods of time, it is difficult to determine when an abnormality will occur and when an abnormal signal will be generated. Accordingly, it is difficult to receive an accurate diagnosis through the above test, which is based on only a short sampling of heart activity.

### SUMMARY

In one general aspect there is provided an apparatus for remotely managing a disease, in particular by monitoring a biosignal, the apparatus including a sensor having a first determining unit configured to determine whether a measured biosignal has a normal waveform, and a transmitter configured to transmit the biosignal in response to the biosignal being determined to have an abnormal waveform; and a server configured to receive the transmitted biosignal.

The server may include a second determining unit configured to determine a type of disease corresponding to the biosignal.

The apparatus for remote management therefore enables automatic transmission of data and diagnosis of the transmitted data.

The biosignal may be an electrocardiogram (ECG), and the second determining unit may be configured to determine a type of arrhythmia corresponding to the ECG.

The second determining unit may automatically determine the type of arrhythmia corresponding to the ECG. This operation may provide classification to support the checking and analyzing operation performed by a medical team.

The apparatus may include a gateway having a display unit, and the gateway may be configured to receive the biosignal from the sensor and transfer the biosignal to the server, and the display unit may be configured to display the biosignal.

The server may be configured to transmit the ECG and arrhythmia type information to a medical team or a medical professional.

The medical team may easily analyze an arrhythmia waveform of an abnormal waveform, and may manage a larger number of patients in a short period of time. This enables greater efficiency and more accurate diagnosis.

The server may be further configured to transmit the biosignal to a medical team or a medical professional, receive a diagnosis result from the medical team or the medical professional, and transmit the diagnosis result to the gateway; the gateway may be further configured to receive the diagnosis result; and the display unit may be further configured to display the diagnosis result.

During regular daily activity, generated ECG data may be transmitted in real time to the server through the gateway that uses wireless transmission technology. The ECG data may be stored in the server. Additionally, the server may automatically analyze a state of a patient using the ECG data, and notify the medical team and the patient of emergency information. All gathered ECG data may be transmitted to the medical team to provide a real-time monitoring of the patient's heart activity. Additionally, feedback is displayed on the display unit so that the patient may perform the appropriate actions.

The first determining unit may be further configured to determine whether the ECG has a normal waveform based on a rhythm and a feature point of an R wave of the ECG.

The transmitter may be further configured to transmit only an interval of the ECG, and the second determining unit may be further configured to determine the type of arrhythmia based on a rhythm and a feature point of an R wave of the transmitted interval.

The transmitter may be further configured to transmit only an interval of the ECG, and the interval may include only a portion of the ECG showing arrhythmia and two minutes before and after that portion.

Thereby, it is possible to efficiently manage a patient having a cardiac disease, by reducing unnecessary data and screening only required information. Since only data required to be analyzed may be transmitted, data transmission may be efficiently managed.

In another general aspect, there is provided a method of remotely managing a disease, in particular by monitoring a biosignal, the method including measuring the biosignal, determining whether the measured biosignal has a normal waveform; transmitting the biosignal in response to the biosignal being determined to have an abnormal waveform; and receiving the transmitted biosignal.

The biosignal may be an electrocardiogram (ECG), and the method may further include determining a type of arrhythmia corresponding to the ECG using a server.

The determining of whether the measured biosignal has a normal waveform may comprise determining whether the ECG has a normal waveform based on a rhythm and a feature point of the ECG.

The determining of whether the measured biosignal has a normal waveform may comprise determining whether the ECG has a normal waveform based on a rhythm and a feature point of an R wave of the ECG.

The transmitting of the biosignal may comprise transmitting only an interval of the ECG, and the determining of the type of arrhythmia may be based on a rhythm and a feature point of the transmitted interval.

The transmitting of the biosignal may comprise transmitting only an interval of the ECG, and the determining of the type of arrhythmia may be based on a rhythm and a feature point of an R wave of the ECG.

The method may further include transmitting arrhythmia type information to a medical team or a medical professional.

The method may further include receiving a diagnosis result from the medical team or the medical professional; and displaying the diagnosis result.

The transmitting of the biosignal may include transmitting only an interval of the ECG; the transmitted interval may include only a portion of the ECG showing arrhythmia and two minutes before and after that portion; and the method may further include displaying the transmitted interval.

Accordingly, arrhythmia classification for individual ECG signals may be accurately performed by repeatedly performing the above process. Furthermore, an amount of power consumed by an ECG sensor unit to monitor a patient for 24 hours may be reduced. Thus, it is possible to use a smaller and more convenient sensor unit. Also, the time and costs required by a medical team to analyze the data may be reduced.

In another general aspect, there is provided a non-transitory computer-readable storage medium storing a program for controlling a computer to execute the method.

In another general aspect, there is provided a wireless sensor for remotely managing a disease, in particular by monitoring a biosignal, the wireless sensor including a first determining unit configured to determine whether a measured biosignal has a normal waveform; and a transmitter configured to wirelessly transmit the biosignal, in response to the biosignal being determined to have an abnormal waveform, to a second determining unit configured to determine a type of the disease.

### BRIEF DESCRIPTION OF THE DRAWINGS

FIG. 1 is a diagram illustrating an example of an apparatus for managing a cardiac disease using a Holter monitor.
FIG. 2 is a diagram illustrating an example of a cardiac disease management system for remote management that enables automatic transmission of data.
FIG. 3 is a diagram illustrating an example of data for management of cardiac diseases.
FIG. 4 is a diagram illustrating an example of a configuration of a cardiac disease management apparatus.
FIG. 5 is a flowchart illustrating an example of a method of managing a cardiac disease.
FIG. 6 is a diagram illustrating an example of an electrocardiogram (ECG) to determine whether a cardiac disease is present.
FIG. 7 is a diagram illustrating an example of a configuration of an apparatus for managing a cardiac disease.

### DETAILED DESCRIPTION

The following detailed description is provided to assist the reader in gaining a comprehensive understanding of the methods, apparatuses, and/or systems described herein. However, various changes, modifications, and equivalents of the systems, apparatuses and/or methods described herein will be apparent to one of ordinary skill in the art. Also, descriptions of functions and constructions that are well known to one of ordinary skill in the art may be omitted for increased clarity and conciseness.

Throughout the drawings and the detailed description, the same reference numerals refer to the same elements. The drawings may not be to scale, and the relative size, proportions, and depiction of elements in the drawings may be exaggerated for clarity, illustration, and convenience.

The features described herein may be embodied in different forms, and are not to be construed as being limited to the examples described herein. Rather, the examples described herein have been provided so that this disclosure will be thorough and complete, and will convey the full scope of the disclosure to one of ordinary skill in the art.

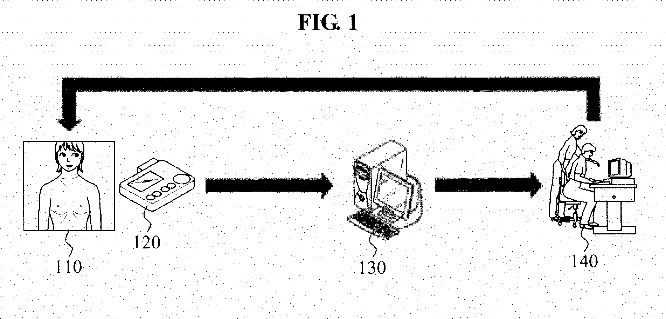

FIG. 1 illustrates an example of an apparatus for managing a cardiac disease using a Holter monitor 120. For example, an apparatus for managing a cardiac disease using a Holter monitor 120 for 24 hours may be configured as illustrated in FIG. 1.

In this example, a Holter monitor 120 may be attached to a patient 110 suspected to have heart abnormalities, and an electrocardiogram (ECG) of the patient 110 may be stored in the Holter monitor 120 for 24 hours during regular daily activity of the patient 110. When the patient 110 visits a hospital and transfers the Holter monitor 120 in which the ECG is stored, a medical team 140 may check all data of the patient 110 using a personal computer (PC) 130 to which the data is downloaded via a universal serial bus (USB) or other storage device. As a result, it may determine whether a heart abnormality is present.

However, a method using the above Holter monitor 120 may lead to the problem of a patient needing to visit a hospital multiple times. Also, effort, time and cost to check the data collected over the 24 hour period may require special attention and effort by an on-site medical team.

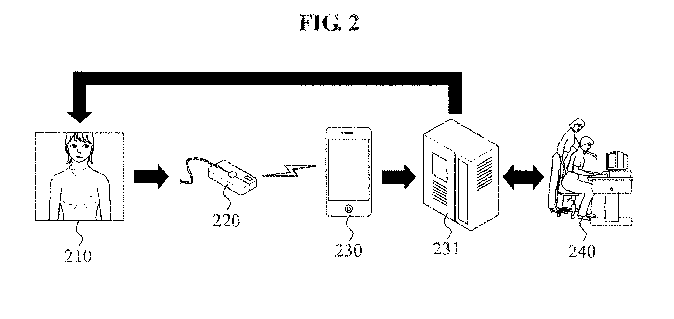

FIG. 2 illustrates an example of a cardiac disease management system for remote management that enables automatic transmission of data and diagnosis of the transmitted data.

Referring to FIG. 2, a sensor 220 may be attached to a patient 210 with a cardiac disease. The sensor 220 may measure data for 24 hours. During regular daily activity, generated ECG data may be transmitted in real time to a server 231 through a terminal 230 that uses wireless transmission technology. The ECG data may be stored in the server 231. Additionally, the server 231 may automatically analyze a state of a patient using the ECG data, and notify a medical team 240 and the patient 210 of emergency information. For example, the server may notify the medical team 240 and the patient 210 when a heart abnormality is detected.

In an example, the server 231 may transmit all gathered ECG data to the medical team 240 without analyzing the state of the patient 210. Accordingly, all gathered ECG data may be transmitted to the medical team 240 to provide a real-time monitoring of the patient's heart activity. In this example, a terminal used by the medical team 240 may process the data and provide alerts based on the state of the patient 210. In another example, the server 231 may transmit analyzed data or all data to another authorized terminal. For example, the server 231 may transmit the ECG data to an authorized family member or caretaker. The data may be encrypted allowing only the authorized user access to the ECG data.

In these examples, since an ECG signal of a patient 210 is transmitted to a server 231 during the 24 hour period, the sensor 220 and the server 231 may be required to communicate with each other at all times. This may lead to a great amount of power consumption by the sensor 220. In this example, a battery capacity of the sensor 220 may also be increased.

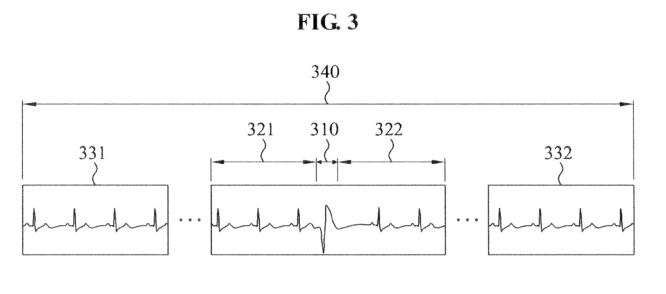

FIG. 3 illustrates an example of data that is collected for management of cardiac diseases. To manage cardiac diseases, for example arrhythmia, data obtained before and after a point in time at which arrhythmia occurs may be analyzed. A type and frequency of a waveform 321 generated before the arrhythmia occurs and a waveform 322 generated after the arrhythmia occurs may be determined, and a condition of a patient may be diagnosed.

As illustrated in FIG. 3, data for management of a cardiac disease may include an abnormal signal in a predetermined interval that is represented by a waveform 310 resulting from arrhythmia of a patient, or an arrhythmia waveform 310, and the waveforms 321 and 322. For example, data including 2 minutes before and after the generation of an arrhythmia waveform 310 may be analyzed; however, a predetermined interval to be analyzed may not be limited thereto.

In an example of a patient with arrhythmia, the arrhythmia may occur at least once, twice, or dozens of times during the waveform 340 corresponding to the 24 hour period. Accordingly, only a portion or portions of the waveform 340 corresponding to abnormal heart activity may require checking by a medical team. In numerous cases, normal waveforms 331 and 332 may not need to be transferred to the medical team, or data transmission may not be required. Thus, it is possible to efficiently manage a patient having a cardiac disease, by reducing the above unnecessary data and screening only required information.

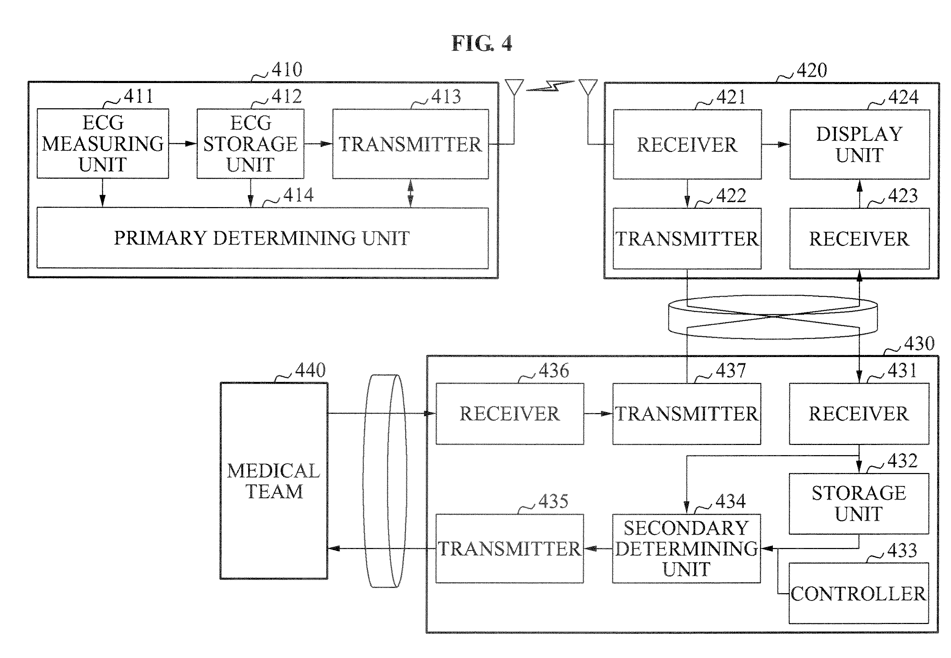

FIG. 4 illustrates an example of a configuration of a cardiac disease management apparatus. Referring to FIG. 4, the disease management apparatus includes an ECG sensor unit 410, a gateway 420, and a server 430.

In this example, the ECG sensor unit 410 measures a normal waveform or an arrhythmia waveform using an ECG measuring unit 411. A signal with the measured waveform is stored in an ECG storage unit 412. In an example, data ranging from about two minutes before or after a point in time at which arrhythmia occurs may be stored in the ECG storage unit 412 after arrhythmia being determined. Additionally, all measured data may be stored in the storage unit 412.

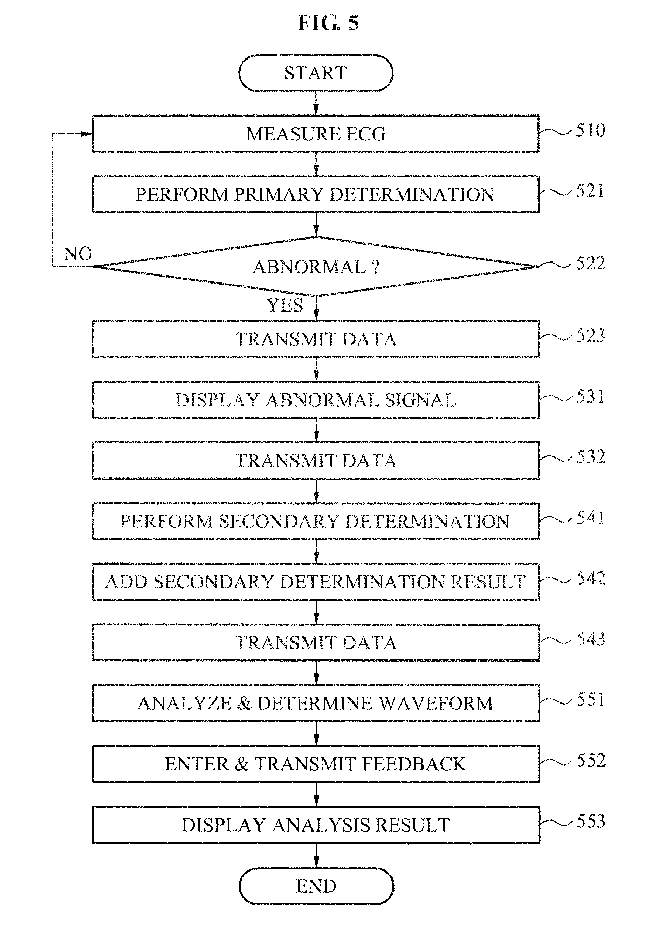

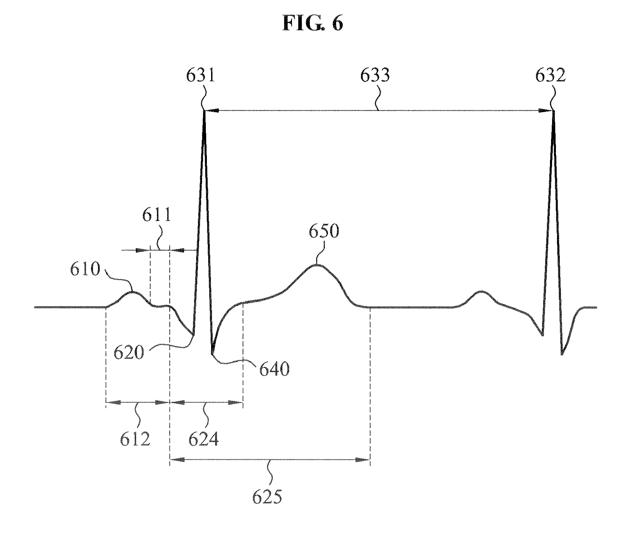

For example, the ECG signal measured by the measuring unit 411 may be stored in the storage unit 412, and whether the measured signal is a normal signal or an abnormal signal may be determined by a primary determining unit 414. Whether the measured ECG signal has a normal waveform or an abnormal waveform may be determined based on rhythms of an ECG using an R waveform and whether a feature point of the measured ECG signal exists. An operation of determining whether the measured ECG signal has a normal or abnormal waveform will be further described with reference to FIGS. 5 and 6.

In this example, when the measured signal is determined to have an abnormal waveform by the primary determining unit 414, a predetermined interval of the signal may be transmitted to the gateway 420 through a transmitter 413. The signal of the predetermined interval ("the abnormal signal") may be the ECG signal determined to have the abnormal waveform by the primary determining unit 414, and may include data corresponding to two minutes before or after the abnormal waveform. The data may be stored in the ECG storage unit 412. Since only data required to be analyzed may be transmitted, data transmission may be efficiently managed.

In this example, the gateway 420 receives the abnormal signal through a receiver 421 of the gateway 420, and displays a waveform on a display unit 424 of the gateway 420 to notify a user of the occurrence of an abnormality. Additionally, the gateway 420 transmits the abnormal signal to the server 430 through a transmitter 422 of the gateway 420.

For example, the server 430 receives an abnormal signal through a receiver 431 of the server 430. The server 430 may be installed in a hospital, or a service organization that performs a cardiac disease management service. In this example, the abnormal signal is stored in a storage unit 432 of the server 430, and controlled by a controller 433 of the server 430. Since the abnormal signal may not include information regarding whether the abnormal signal corresponds to arrhythmia, or information regarding a type of arrhythmia, a medical team may be required to check and analyze all abnormal signals. Accordingly, a large amount of time and effort may be required to check and analyze data transmitted from a large number of patients with cardiac diseases. To prevent such a waste of resources, a secondary determining unit 434 of the server 430 performs an operation that will be described below.

In this example, the secondary determining unit 434 determines whether the input abnormal signal actually represents an arrhythmia waveform, based on a feature point and a rhythm of the input abnormal signal. Additionally, when the input abnormal signal is determined to represent the arrhythmia waveform, the secondary determining unit 434 may automatically determine the type of arrhythmia corresponding to the input signal. The above operation may provide classification to support the checking and analyzing operation performed by the medical team. It should be appreciated that the primary determining unit 414 and the secondary determining unit 434 may also be referred to as the first determining unit 414 and the second determining unit 434. An example of an operation for determining a type of arrhythmia will be further described with reference to FIG. 6.

In this example, information regarding the type of arrhythmia, as obtained by the secondary determining unit 434, is transmitted or displayed to a medical team 440 together with the abnormal signal. The information and signal are transmitted through a transmitter 435 of the server 430. The medical team 440 diagnoses an abnormal signal based on the arrhythmia type information that is transmitted or displayed. In an example, in which arrhythmia type information does not exist, the medical team 440 may require a large amount of time and effort to analyze the type of arrhythmia. In another example, in which the arrhythmia type information is displayed, the arrhythmia type information may be helpful in analyzing the type of arrhythmia. Accordingly, the medical team 440 may easily analyze an arrhythmia waveform of an abnormal signal, and may manage a larger number of patients in a short period of time. This enables greater efficiency and more accurate diagnosis.

In this example, once arrhythmia diagnosis is performed, the medical team 440 provides feedback according to a condition of the patient based on the analyzed data. If serious arrhythmia is diagnosed, the medical team 440 may request a patient to visit a hospital. If only slight arrhythmia is diagnosed, the medical team 440 may alarm a patient to be careful. The medical team 440 transmits analysis results as feedback to a receiver 423 of the gateway 420, through a receiver 436 and transmitter 437 of the server 430. Additionally, feedback is displayed on the display unit 424 so that the patient may perform the appropriate actions.

FIG. 5 illustrates an example of a method of managing a cardiac disease.

Referring to FIG. 5, in 510, an ECG signal of a patient with a cardiac disease is measured. In operation 521, whether the measured ECG signal is a normal signal or an abnormal signal is determined by a processor. In an example, in which the measured ECG signal is determined to be an abnormal signal in operation 522, data of the abnormal signal corresponding to a predetermined interval before and after arrhythmia occurs is transmitted to a gateway in operation 523.

For example, in operation 521, a heart rate may be calculated by calculating the interval between waveforms of the signal. Also, arrhythmia may be diagnosed based on the shape of a single extracted waveform of the signal. In the example illustrated in FIG. 6, the single extracted waveform of the ECG signal includes five peaks protruding from an isoelectric line. These include a P waveform 610, a Q waveform 620, R waveforms 631 and 632, an S waveform 640, and a T waveform 650 as illustrated in FIG. 6. A single waveform is generated every time a heart contracts. The R waveforms 631 and 632, having the largest amplitude, may be detected from among the other waveforms, and a single waveform of the signal may be extracted.

In operation 531, the abnormal signal is displayed to a user by the gateway. In operation 532, data including the abnormal data is transmitted to a server.

In this example, secondary determination and transmission of data to a medical team may be performed in operations 541-543. In operation 541, the processor determines whether the abnormal signal transmitted to the server actually represents an abnormal waveform and determines a type of arrhythmia in the abnormal waveform. In operation 542, the arrhythmia type information obtained by operation 541 is synchronized with the abnormal signal for data transmission, and in operation 543 the data is transmitted to the medical team.

In this example, the medical team analyzes and diagnoses a waveform of the abnormal signal using the received abnormal signal and the received arrhythmia type information in operation 551. In operation 552, the medical team enters a feedback based on a diagnosis result and transmits the feedback to the server and the gateway, and in operation 533, a diagnosed analysis result is displayed to the user in the gateway so that the user performs an action based on the diagnosis result.

FIG. 6 illustrates an example of an ECG to determine whether a cardiac disease is present. Referring to FIG. 6, a single waveform of an ECG includes, for example, the P waveform 610, the Q waveform 620, the R waveforms 631 and 632, the S waveform 640, and the T waveform 650.

In this example, a secondary determination, performed by a secondary determining unit of a server, determines whether an abnormal signal indicates arrhythmia using an artificial neural network. Also, a type of arrhythmia of the abnormal signal may be determined.

An artificial neural network represents an operation model implemented by software or hardware that imitates a calculation capacity of a biological system using a large number of artificial neurons connected by a connection line. In the artificial neural network of this example, artificial neurons obtained by simplifying functions of biological neurons may be used. Additionally, artificial neurons may be interconnected via connection lines having connection strengths. Accordingly, recognition and learning processes of humans may be performed. The connection strength may be a feature value of a connection line, and may be referred to as a connection weight. The artificial neural network may be divided into supervised learning and unsupervised learning.

In supervised learning, input data and corresponding output data are input to the neural network, and the connection strength of connection lines are updated so that the output data is output. A typical learning algorithm may include, for example, a delta rule and backpropagation learning.

In unsupervised learning, an artificial neural network learns connection strengths using only input data without a desired value. In this example, connection weights may be updated by correlation between input patterns.

In an example, pattern classification schemes may be applied to a medical device for determining a disease. For example, electrical body signals of a patient input through the medical device are measured, and patterns of the measured signals are classified for determining a disease. The electrical body signals may include, for example, an ECG, an electroencephalogram (EEG), an electromyogram (EMG), and the like.

In this example, features of an ECG signal may be extracted from an input abnormal signal. The ECG signal may be used to determine whether abnormalities of a conduction system from a heart to an electrode occur and whether a disease exists.

Referring to FIG. 6, feature points extracted from the ECG signal include the heart rate, the QRS duration 624, the PR interval 612, the QT interval 625, a type of a T waveform, and the like. The feature points of the signal may be periodically extracted, and the heart rate, the QRS duration 624, the PR interval 612, the QT interval 625, and the type of the T waveform may be morphometric characteristics of the ECG signal.

In this example, the peaks of the R waveforms 631 and 632 are determined by extracting the maximum voltage values at regular intervals. Two points at which the R waveforms intersect a baseline are extracted to determine the beginning and end points of the QRS wave complex. The point having the largest voltage value on the left side of the QRS complex is extracted to determine the peak of the P waveform 610. A value having a large difference with the baseline between a maximum value and a minimum value on the right side of the QRS complex is extracted to determine a peak of the T waveform 650. A point on the baseline to the left of the peak of the P waveform is extracted to determine a beginning point of the P waveform, and a point on baseline to the right of the peak of the T waveform is extracted determine an end point of the T waveform.

Additionally, points having the lowest voltage values on the left and the right side of the peak of the R waveform 631 are extracted to determine a peak of the Q waveform 620 and a peak of the S waveform 640, respectively. A point at which the signal intersects the baseline for the first time on the left side of the peak of the Q waveform 620 is extracted to determine a beginning point of the Q waveform 620. A point at which the signal intersects the baseline for the first time on the right side of the peak of the S waveform 640 is extracted to determine an end point of the S waveform 640. A PR interval may be obtained by determining the distance from the beginning point of the P waveform to the beginning point of the QRS complex, and a QT interval may be obtained by determining a distance from the beginning point of the QRS complex to an end point of the T waveform. Also, a QRS duration is obtained by determining the distance from the beginning point of the Q waveform to the end point of the S waveform. The heart rate of a patient may be used to recognize the number of times basic waveforms as shown in FIG. 6 exist per minute. Additionally, the type of the T waveform may be used to indicate a direction of the peak of the T waveform.

In this example, the secondary determining unit extracts the feature points using morphometric characteristics of the ECG signal. In a normal ECG signal, a PR interval is typically 0.6 s to 1 s. In this example, an R waveform is found every 0.8 s. For some types of arrhythmia, such as arteria and bradyrhythmia, an R waveform may not be generated at all for some intervals. Accordingly, when a voltage peak is found every 0.8 s, a peak voltage for an interval in which a normal R waveform is not actually present may be incorrectly determined as an R waveform. Therefore, for this example, a predetermined threshold may be applied so that if the magnitude of an extracted R waveform does not exceed the predetermined threshold, the extracted R waveform may not be recognized as an R waveform. A scheme of extracting the R waveform may be used to determine whether a measured ECG signal is a normal signal or an abnormal signal by a primary determining unit.

In an example, the Q and S waveforms are also extracted. Based on a peak of an extracted R waveform, the peak of the Q waveform and the peak of the S waveform may have lowest voltage values on the left side and the right side of the R waveform, respectively. Based on a type of signal, a Q waveform and an S waveform may not exist, and only an R waveform may be found to exist. In that example, the two points in which the R waveform intersects the baseline are determined to be the beginning and end points of the QRS complex. When the Q and S waveforms are found, a distance from a beginning point of the Q waveform to an end point of the S waveform may be obtained, and may correspond to a QRS duration. In this example, when a QRS duration is equal to or longer than 0.12 s, an abnormal QRS duration is detected and right bundle branch block (RBBB) or left bundle branch block (LBBB) may be diagnosed.

In an example, the P and T waveforms are also extracted. A curve of the P waveform has a peak pointing upward, thus the peak corresponds to the point with the largest voltage value on the left side of the beginning point of the QRS complex. A beginning point and an end point of the P waveform may correspond to points on the baseline to the left side and the right side of the peak of the P waveform, respectively. In the T waveform, a peak of a normal waveform points upward. However, in a curve of an LBBB, a peak may point downward. Accordingly, the peak of the T waveform may correspond to a value with a large difference between a maximum value and a minimum value on the right side of the end point of the QRS complex. Two points meeting the baseline may be found from the left and right sides of the extracted point, and accordingly a beginning point and an end point of the T waveform may be found. When the P waveform and the T waveform are found, a PR interval may be obtained using a distance from the beginning point of the P waveform to the beginning point of the QRS complex, and a QT interval may be obtained using a distance from the beginning point of the QRS complex to the end point of the T waveform.

The extracted ECG feature points and corresponding data may be input to the artificial neural network. A type of arrhythmia may be determined based on an output value of the artificial neural network.

For example, a secondary determining unit may perform learning of the artificial neural network, using ECG data received from an ECG-MIT-BIH database. The ECG-MIT-BIH DB is a database of electrocardiography data used to diagnose arrhythmia. That is, the ECG-MIT-BIH DB is a database of electrocardiography signals obtained from the human body having a particular type of arrhythmia, and stores correspondence between electrocardiography signals and types of arrhythmia. In this example, the ECG-MIT-BIH DB is ECG data used to diagnose arrhythmia. The ECG-MIT-BIH DB may represent ECG signals acquired from a human body with a predetermined arrhythmia. When such an ECG signal is represented, a type of arrhythmia may be stored.

The ECG data received from the ECG-MIT-BIH DB may be information regarding an ECG signal measured from a plurality of human bodies, and accordingly both input data and an output value may be known. For example, the input data may be values extracted from the ECG signal, and the output value may be a value based on the type of arrhythmia. Accordingly, since an output value of input ECG data may be known in advance, the learning of the artificial neural network may correspond to supervised leaning. Therefore, the artificial neural network may be taught using ECG data received from the ECG-MIT-BIH DB, and connection strengths may be determined in advance.

Additionally, the secondary determining unit may receive data corresponding to the extracted feature points, and may determine the type of arrhythmia based on the received data. The type of arrhythmia may be determined using an output value of the artificial neural network.

In this example, connection strengths of predetermined connection lines may be updated using the extracted data and output value. An output value may be assumed to be a result value indicating a determined type of arrhythmia, and connection strengths of predetermined connection lines may be updated. Since connection strengths of main components of the network may be updated using an individual ECG signal, the network may have connection strengths that are optimized to a particular individual. Since signals may be classified based on connection strengths optimized to an individual, classification of individual arrhythmia may clearly be performed. Accordingly, based on an individual signal, an individual may check the type of arrhythmia, for example an atrial fibrillation, an atrial flutter, a ventricular tachycardia, and the like.

For example, when a value [0.89, 0.77] is output in response to an input of first data extracted from feature points, the output value [0.89, 0.77] may correspond to atrial fibrillation. Additionally, a result value indicating the atrial fibrillation may be [1, 1]. In this example, the secondary determining unit may input the first data to an input layer of the artificial neural network, and update the connection strengths of predetermined connection lines so that [1, 1] is the output value. The above process may enable the artificial neural network to have connection strengths suitable for individual ECG signals. Accordingly, arrhythmia classification for individual ECG signals may be accurately performed by repeatedly performing the above process.

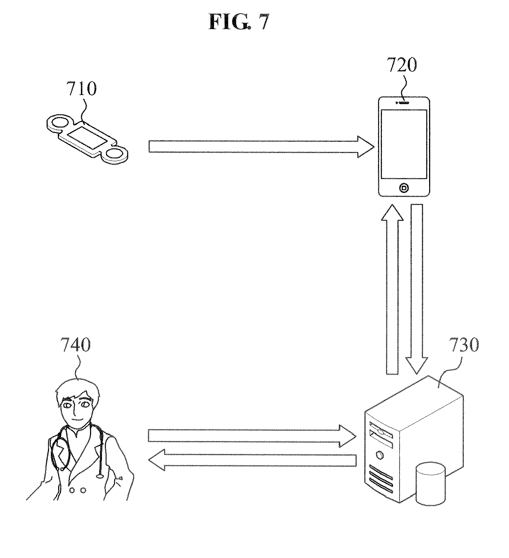

FIG. 7 illustrates an example of a configuration of an apparatus for managing a cardiac disease. An amount of power consumed by an ECG sensor unit 710 to monitor a patient for 24 hours may be reduced. Also, the time and costs required by a medical team to analyze data may be reduced.

In this example, whether an ECG signal is a normal signal is primarily analyzed by an ECG sensor unit 710. When the ECG signal is determined to be an abnormal signal, a predetermined interval of the signal including a point in which arrhythmia occurs may be transmitted to a server 730 through a gateway 720. In this example, the gateway 720 may wirelessly communicate with the ECG sensor unit 710.

The server 730 secondarily analyzes the abnormal signal, and determines whether arrhythmia occurs. When arrhythmia is determined to occur, a type of arrhythmia will automatically be determined. Accordingly, data combining the abnormal signal and arrhythmia type information may be transmitted to a medical team 740. The medical team 740 may provide, as feedback, a result obtained by diagnosing the abnormal signal based on the arrhythmia type information. This result is transmitted to the gateway 720 via the server 730.

To efficiently monitor the ECG signal over a 24 hour period, the sensor 710 may primarily analyze the ECG of the patient and determine whether the patient is in normal or abnormal condition. Based on this analysis, the sensor 710 may transmit data to the server 730 only in cases of necessity; for example, when an abnormal condition is detected. Thus, it is possible to reduce power consumption of the sensor 710, allowing for the use of a smaller sized and more convenient sensor 710.

Additionally, the abnormal signal to be analyzed by the medical team 740 through secondary analysis may automatically be analyzed by determining the type of arrhythmia and displaying the results to the medical team 740. Accordingly, useful information is provided to the medical team 740 to ultimately determine a condition of the patient. The time and costs required by the medical team to analyze the data is reduced, and the medical team may more accurately analyze the ECG signal. Therefore, it is possible to effectively manage a cardiac disease.

While the apparatus and method have been described for remotely managing a patient with a cardiac disease such as arrhythmia, other cardiac diseases may be managed based on the monitored ECG signal. Also, the apparatus and method may be used for monitoring and managing patients having other diseases by monitoring other types of biosignals. For example, musculoskeletal diseases may be managed by monitoring Electromyogram signals (EMG). Similarly, diseases related to brain activity may be managed by monitoring Electroencephalogram signals (EEG) or Magnetoencephalogram (MEG) signals. Also, Electrooculography (EOG) may be used to monitor ocular diseases and Galvanic skin response (GSR) signals may be used to monitor skin diseases.

The Holter monitor, personal computers, sensors, terminals, gateways, and servers described above may be implemented using one or more hardware components, or a combination of one or more hardware components and one or more software components. A hardware component may be, for example, a physical device that physically performs one or more operations, but is not limited thereto. Examples of hardware components include controllers, microphones, amplifiers, low-pass filters, high-pass filters, band-pass filters, analog-to-digital converters, digital-to-analog converters, and processing devices.

A processing device may be implemented using one or more general-purpose or special-purpose computers, such as, for example, a processor, a controller and an arithmetic logic unit, a digital signal processor, a microcomputer, a field-programmable array, a programmable logic unit, a microprocessor, or any other device capable of running software or executing instructions. The processing device may run an operating system (OS), and may run one or more software applications that operate under the OS. The processing device may access, store, manipulate, process, and create data when running the software or executing the instructions. For simplicity, the singular term "processing device" may be used in the description, but one of ordinary skill in the art will appreciate that a processing device may include multiple processing elements and multiple types of processing elements. For example, a processing device may include one or more processors, or one or more processors and one or more controllers. In addition, different processing configurations are possible, such as parallel processors or multi-core processors.

Software or instructions for controlling a processing device, such as those described in FIGS. 5 and 6, to implement a software component may include a computer program, a piece of code, an instruction, or some combination thereof, for independently or collectively instructing or configuring the processing device to perform one or more desired operations. The software or instructions may include machine code that may be directly executed by the processing device, such as machine code produced by a compiler, and/or higher-level code that may be executed by the processing device using an interpreter. The software or instructions and any associated data, data files, and data structures may be embodied permanently or temporarily in any type of machine, component, physical or virtual equipment, computer storage medium or device, or a propagated signal wave capable of providing instructions or data to or being interpreted by the processing device. The software or instructions and any associated data, data files, and data structures also may be distributed over network-coupled computer systems so that the software or instructions and any associated data, data files, and data structures are stored and executed in a distributed fashion.

For example, the software or instructions and any associated data, data files, and data structures may be recorded, stored, or fixed in one or more non-transitory computer-readable storage media. A non-transitory computer-readable storage medium may be any data storage device that is capable of storing the software or instructions and any associated data, data files, and data structures so that they can be read by a computer system or processing device. Examples of a non-transitory computer-readable storage medium include read-only memory (ROM), random-access memory (RAM), flash memory, CD-ROMs, CD-Rs, CD+Rs, CD-RWs, CD+RWs, DVD-ROMs, DVD-Rs, DVD+Rs, DVD-RWs, DVD+RWs, DVD-RAMs, BD-ROMs, BD-Rs, BD-R LTHs, BD-REs, magnetic tapes, floppy disks, magnetooptical data storage devices, optical data storage devices, hard disks, solid-state disks, or any other non-transitory computer-readable storage medium known to one of ordinary skill in the art.

Functional programs, codes, and code segments for implementing the examples disclosed herein can be easily constructed by a programmer skilled in the art to which the examples pertain based on the drawings and their corresponding descriptions as provided herein.

While this disclosure includes specific examples, it will be apparent to one of ordinary skill in the art that various changes in form and details may be made in these examples without departing from the scope of the claims and their equivalents. The examples described herein are to be considered in a descriptive sense only, and not for purposes of limitation. Descriptions of features or aspects in each example are to be considered as being applicable to similar features or aspects in other examples. Suitable results may be achieved if the described techniques are performed in a different order, and/or if components in a described system, architecture, device, or circuit are combined in a different manner and/or replaced or supplemented by other components or their equivalents. Therefore, the scope of the disclosure is not defined by the detailed description, but by the claims and their equivalents, and all variations within the scope of the claims and their equivalents are to be construed as being included in the disclosure.

## Claims

1. An apparatus for remotely monitoring a biosignal, in particular an electrocardiogram (ECG), the apparatus comprising:
a sensor comprising a first determining unit configured to determine whether a measured biosignal has a normal waveform, and a transmitter configured to transmit the biosignal in response to the biosignal being determined to have an abnormal waveform; and
a server configured to receive the transmitted biosignal.

2. The apparatus of claim 1, wherein the server comprises a second determining unit configured to determine a type of disease, in particular a type of arrhythmia, corresponding to the biosignal.

3. The apparatus of claim 1 or 2, further comprising:
a gateway comprising a display unit, wherein the gateway is configured to receive the biosignal from the sensor and transfer the biosignal to the server, and the display unit is configured to display the biosignal.

4. The apparatus of one of claims 1 to 3, wherein the server is configured to transmit the biosignal, in particular the ECG and arrhythmia type information, to a medical team or a medical professional.

5. The apparatus of claim 4, wherein:
the server is further configured to receive a diagnosis result from the medical team or the medical professional, and transmit the diagnosis result to the gateway;
the gateway is further configured to receive the diagnosis result; and
the display unit is further configured to display the diagnosis result.

6. The apparatus of claim 1 or 2, wherein the first determining unit is further configured to determine whether the ECG has a normal waveform based on a rhythm and a feature point of an R wave of the ECG.

7. The apparatus of claim 2, wherein the transmitter is further configured to transmit only an interval of the ECG, and the second determining unit is further configured to determine the type of arrhythmia based on a rhythm and a feature point of an R wave of the transmitted interval.

8. The apparatus of claim 2, wherein the transmitter is further configured to transmit only an interval of the ECG, and the interval includes only a portion of the ECG showing arrhythmia and two minutes before and after that portion.

9. A method of remotely monitoring a biosignal, the method comprising:
determining whether a measured biosignal has a normal waveform;
transmitting the biosignal in response to the biosignal being determined to have an abnormal waveform; and
receiving the transmitted biosignal.

10. The method of claim 9, wherein the biosignal is an electrocardiogram (ECG), and the method further comprises determining a type of arrhythmia corresponding to the ECG using a server or determining a type of arrhythmia corresponding to the ECG using a server and transmitting arrhythmia type information to a medical team or a medical professional.

11. The method of claim 10, wherein the determining of whether the measured biosignal has a normal waveform comprises determining whether the ECG has a normal waveform based on a rhythm and a feature point, in particular of an R wave, of the ECG.

12. The method of claim 10, wherein the transmitting of the biosignal comprises transmitting only an interval of the ECG, and the determining of the type of arrhythmia is based on a rhythm and a feature point, in particular of an R wave, of the ECG.

13. The method of one of claims 10 to 12, further comprising:
receiving a diagnosis result from the medical team or the medical professional; and
displaying the diagnosis result.

14. The method of one of claims 10 to 13, wherein:
the transmitting of the biosignal comprises transmitting only an interval of the ECG;
the transmitted interval includes only a portion of the ECG showing arrhythmia and two minutes before and after that portion; and
the method further comprises displaying the transmitted interval.

15. A non-transitory computer-readable storage medium storing a program for controlling a computer to execute the method of one of claims 10 to 14.
